# EUROPEAN PATENT APPLICATION

(11) **EP 2 179 691 A1**
(43) Date of publication of application: **28.04.2010**
(21) Application number: 08396016.1
(22) Date of filing: 23.10.2008
(51) Int. Cl.: A61B 5/083, G01N 33/497

(54) **Gas analyzing unit and airway adapter**

(71) Applicant: General Electric Company, Schenectady, NY 12345 (US)
(72) Inventor: Haveri, Heikki, 03150 Huhmari (FI)
(74) Representative: Kanerva, Arto

(57) **Abstract**

An airway adapter is disclosed herein. The airway adapter includes a sampling chamber (31) for allowing a respiratory gas flow. The airway adapter also includes a sensor (40) for acquiring a signal needed for a respiratory gas analysis. The sensor (40) is integrated into the airway adapter and which sensor comprises an electrolyte being in contact with the respiratory gas flowing in the sampling chamber and which electrolyte contact with the respiratory gas is a basis for respiratory gas analysis.

## Description

### BACKGROUND OF THE INVENTION

This disclosure relates generally to a gas analyzing unit and an airway adapter usable in the gas analyzing unit.

During anesthesia or in critical care, patients are often mechanically ventilated instead of breathing spontaneously. The patient is connected to a patient circuit of a ventilator or an anesthesia machine by intubation: inserting an endotracheal tube to a trachea so that a gas can flow trough it into and out of a lung. The breathing circuit typically consists of the endotracheal tube, a Y-piece where inspiratory and expiratory tubes from the ventilator come together, as well as the ventilator.

It is important that the ventilation meets the patient's needs for a correct exchange and administration of oxygen (O₂), carbon dioxide (CO₂) nitrous oxide (N₂O), anesthetic agents and other gases. It is also very important to feed gases at pressures that do not damage the patient's lung. The gases should also be given at a suitable respiration rate and tidal volume. Anesthesiologists, respiratory therapists and other qualified clinicians use their professional skills to set ventilation parameters to optimally meet needs of the patient. The ventilation is often monitored with a respiratory monitor by measuring essentially in real time concentrations of oxygen, carbon dioxide, nitrous oxide and anesthetic agent in a breathing gas. The respiratory monitor often also have spirometric measurements for monitoring a pressure and gas flow in the patient circuit. From a gas concentration and gas flow data, it is possible to calculate the patient's consumption of oxygen and production of CO₂.

Recent developments in miniaturization of gas sensors have opened the possibility to measure all respiratory gases such as for example CO₂ N₂O, Oxygen and anesthetic agents with a mainstream gas sensor or combination of sensors, placed on an airway adapter in the patient circuit of the ventilator or an anesthesia machine. Mainstream gas sensors transform the measured concentrations to electric signals that are transmitted to the respiratory monitor. This is usually done with a cable that also feeds an operating power from the respiratory monitor to the sensor.

The existing mainstream gas analyzing technologies used for measuring the content of oxygen, or other gases as well, in the breathing gas utilize techniques such as electro-chemical process in a form of for example galvanic-, polarographic- and fuel cells etc., or such as opto-chemical processes in a form of for example sensor cells based on a detection of fluorescence quenching etc. All electro-chemical sensor cells described above have some sort of reactive surface that has to be in contact with the analyzed gas molecules flowing within the breathing gas inside the breathing circuit. Some properties of electro-chemical cells change in time, but the change can be offset calibrated and compensated, which makes the analyzing technique suitable for the small, robust gas measurement. Opto-chemical sensor cells need optical window for a radiation to traverse through the measured gas into the fluorescence surface. Opto-chemical cells are sensitive to ambient changes such as a temperature, humidity etc. A sensitiveness of the fluorescence surface changes strongly in time, thus the measurement needs frequent offset and gain calibration to function properly. Due to this reason opto-chemical cells are unsuitable for small sized, robust gas analyzing in general.

At the reactive surface of the electro-chemical sensor cells oxygen molecules cause a chemical reaction proportional to oxygen content in the gas mixture, which is then converted into an electrical signal. A lifetime of the cell is typically specified as hours in 100% oxygen, which typically is one or several months in normal room air containing approximately 21% of oxygen. In practice the longer lifetime is desired since chemical cells are expensive as they are primarily hand made and it saves time used for configuring the device if it can be done less frequently. The lifetime of the cell is primarily determined by an amount of a chemical or electrolyte stored inside the sensor, but also by the amount of a deposit formed during the measurement process, which decelerates and finally quenches the measurement based on the chemical reaction with oxygen. Quenching of the chemical reaction also initiates different kind of long-term measurement signal offset and gain drifts that cause continuous need for zeroing and/or calibration oftentimes during the sensor cell lifetime. Since a longer lifetime is desired the conventional chemical and electro-chemical sensor cells are much too big and heavy for the mainstream use. Furthermore the storage time and the storage conditions usually degrease the usage time. Cells are also difficult to handle and to place inside the conventional mainstream gas analyzer. New cells may also need additional calibration, but at least zeroing in the use oftentimes so that the measurement functions properly.

In the mainstream use the sensor cell has to be in contact with the breathing gas flowing inside the breathing circuit somehow, so that the analyzed gas molecules can move into the reactive surface and inner parts of the sensor cell. Conventional airway adapter, which is connected to a conventional mainstream analyzer body, provides a passage for the breathing gases to pass from the breathing circuit into the reactive surface of the sensor cell. The passage through the airway adapter in to the mainstream body is typically an open cavity comprising some short of sealing and a porous membrane. Sealing is used between the sensor cell and the airway adapter to prevent a breathing circuit leakage. The leakage mixes the operation of the ventilator since the breathing circuit pressure and flow does not meet a required setting, which then decreases the gas exchange of the patient. Sealing is usually complicated and brakes down easily since the airway adapter is changed back and forth into the gas analyzer body daily or between different patients. The porous membrane or similar with a correct pore size, lets gas molecules, such as oxygen, to traverse through the membrane, but prevents moisture, bacteria etc. to traverse from the breathing circuit in to the non-disposable parts of the analyzer that are difficult to clean, but also from the analyzer into the breathing circuit thus to prevent the spreading of bacteria between different patients and contaminated non-disposable sensor parts.

In practice the porous membrane that gas molecules can pass through properly does not block all the smallest viruses or new viral forms. On the other hand very dense membrane with very small openings increases the sensor response time. A construction to arrange appropriate sealing and appropriate porous membrane between the re-connectable airway adapter and the sensor cell inside the mainstream body increases a length as well as a dead space of the cavity. As there is no gas flow through the cavity, but gas molecules diffuse through the cavity and through the porous membrane finally reaching the sensor cell, the time constant of the measurement and respectively the transient response time is increased considerably, thus limiting the accuracy of the gas analyzing at higher respiration rates. Thus when the gas concentration changes in the breathing circuit, as the patient is ventilated, it takes certain time for the gas molecules to reach the active surface of the sensor cell at the other end of the cavity inside the conventional mainstream analyzer body to cause reaction proportional to the number of gas molecules. At lower respiration rates (RR) gas molecules may reach the sensor cell before the gas concentration in the breathing circuit changes, but at higher RR the gas concentration changes before the gas molecules have even reached the sensor cell, which degrades the measurement as the measured concentration never reach the level of the concentration of the breathing gas flowing in the breathing circuit. Usually the maximum respiration rates that can be reliably measured with inspired/expired ratio (I:E ratio) of 1:2 are well bellow 30.

RR can be determined as terms of rise time Xᵣ = Yᵣ + Zᵣ and fall time X_{f} = Y_{f} + Z_{f}, in which both times include time portions caused by the rise time Yᵣ and the fall time Y_{f} of the sensor cell and the rise time Zᵣ and the fall time Z_{f} of the molecule diffusion through the cavity. The rise time describes how fast the sensor reacts to the gas concentration change in the breathing gas, whereas the fall time describes how fast it recovers from that change as the breathing gas returns into the previous level. The rise time is the time in which the amplitude of the measured signal rises between the 10% and 90% levels of the actual signal. Similarly the fall time is the time in which the amplitude of the measured signal falls between the 10% and 90% levels of the actual signal. In the terms of rise- or fall times RR of 30 with I:E ratio 1:2 means a maximum rise time Xᵣ of 600ms and a maximum fall time X_{f} of 1300 ms to get reliable samples of the flowing gas to generate an electrical signal and further for example corresponding values proportional to the minimum gas concentration during inspiration and the maximum gas concentration during expiration. The rise time Yᵣ and the fall time Y_{f} of the sensor depend on the type of the sensor, but for example for a chemical cell type sensor the both times are usually more than 300 ms. In many cases the molecule diffusion through the cavity is thus much dominant as in this example the rise time Zᵣ of molecule diffusion is more than 300 ms and/or the fall time Z_{f} more than 1000 ms.

To improve the measurement it is possible to add different kind of constructions such as wings etc. inside the airway adapter to guide the breathing gas flow towards the cavity so to improve and to make faster the diffusion of gas molecules through the cavity. Usually these complicated constructions degrade the functioning of the airway adapter and make it sensitive to mucus etc. flowing from the patient.

### BRIEF DESCRIPTION OF THE INVENTION

The above-mentioned shortcomings, disadvantages and problems are addressed herein which will be understood by reading and understanding the following specification.

In an embodiment an airway adapter includes a sampling chamber for allowing a respiratory gas flow and a sensor for acquiring a signal needed for respiratory gas analysis. The sensor is integrated into the airway adapter and which sensor comprises an electrolyte being in contact with the respiratory gas flowing in the sampling chamber and which electrolyte contact with the respiratory gas is a basis for respiratory gas analysis.

In another embodiment, a gas analysis unit for respiratory gas analysis includes an airway adapter having a sampling chamber for allowing a respiratory gas flow and a sensor for acquiring a signal relative to the respiratory gas flowing through the sampling chamber of the airway adapter. The gas analysis unit also includes a processor for analyzing the signal received from the sensor. The sensor is integrated into the airway adapter and the sensor comprises an electrolyte being in contact with the respiratory gas in the sampling chamber and which electrolyte contact with the respiratory gas is a basis for the respiratory gas analysis.

In yet another embodiment, a gas analysis unit for respiratory gas analysis includes an airway adapter having a sampling chamber for allowing a respiratory gas flow and at least one optical window for an optical measurement of the respiratory gas. The gas analysis unit also includes a sensor for acquiring a signal indicative of one of a concentration of a component and an identification of a component of the respiratory gas flowing through the sampling chamber of the airway adapter. The gas analysis unit further includes a gas analyzer for receiving the signal from the sensor, the gas analyzer including a coupling point for an airway adapter, an infrared emitter for emitting an infrared radiation through the sampling chamber and a detector for receiving the infrared radiation and creating an infrared absorption signal and a processor for analyzing the signal from the sensor and the infrared absorption signal from the detector. The sensor is integrated into the airway adapter and which sensor includes an electrolyte being in contact with the respiratory gas in the sampling chamber and which electrolyte contact with the respiratory gas is a basis for respiratory gas analysis.

Various other features, objects, and advantages of the invention will be made apparent to those skilled in art from the accompanying drawings and detailed description thereof.

### BRIEF DESCRIPTION OF THE DRAWINGS

Figure 1 is a schematic perspective view of a gas analyzing unit incorporating an airway adapter in accordance with an embodiment in an operating environment;

Figure 2 is a schematic perspective view of a gas analyzing unit incorporating an airway adapter in accordance with another embodiment in an operating environment; and

Figure 3 is a schematic side view of the airway adapter shown in Figure 1.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

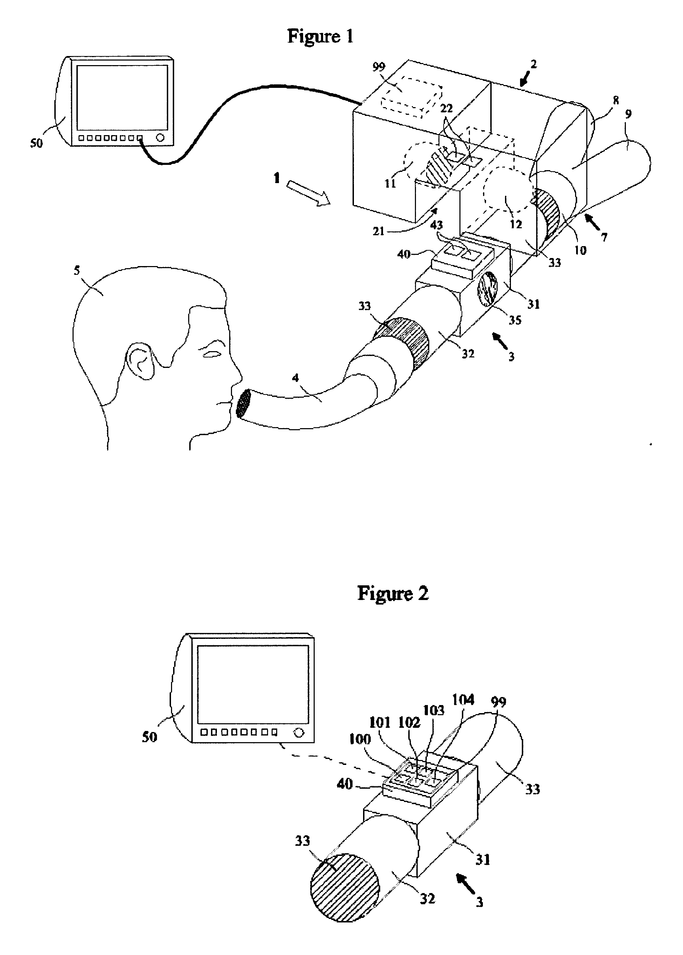

Figure 1 shows a gas analyzing unit 1 comprising a gas analyzer 2 such as a main stream gas analyzer for making one of a qualitative or quantitative gas analysis of at least one respiratory gas like CO₂ or Oxygen. and an airway adapter 3 in a typical operating environment where the gas analyzing unit 1 is connected through an endotracheal tube 4 to airways of a subject 5. The gas analyzing unit 1 may also be connected to a branching unit 7 such as a Y-piece having at least three limbs, one of them being an inhalation limb 8 for inspired gas, a second one being an expiration limb 9 for expired gas, a third one being a combined inspiration and expiration limb 10 for both inspired and expired gases. The inhalation limb 8 is connectable to the inspiration tube (not shown) and the expiration limb 9 is connectable to the expiration tube (not shown), which both tubes may be connected to a ventilator (not shown) for allowing the gas exchange with the airways of the subject 5. The combined inspiration and expiration limb 10 is typically connected to the airway adapter 3.

The gas analyzing unit 1 and its airway adapter 3 connectable between the endotracheal tube 4 and the branching unit 7 as shown in Figure 1 is especially designed for a mainstream measurement whereby a gas measurement is made directly in the main gas flow of the subject 5 and thus this can be done without taking samples out of this main gas flow. The airway adapter 3 includes a sampling chamber 31 between a connector 32 and a connector 33. The connector 32, which can be a female type, and the connector 33, which can be a male type, are used to connect the airway adapter 3 as well as the gas analyzer 2 between the endotracheal tube 4 and the branching unit 7. The inner diameter of the conventional, slightly conical female connector 32 is typically 13-13.5 mm and the length 17-28 mm. The male connectors 33 fit on male connectors in every connection of the breathing circuit, thus the inner diameter is typically approximately from 14.5 to 15.5 mm and the length 17-28 mm. The sampling chamber 31 is not limited to any special form but may be e.g. a tube or a part of tube or it may be a conventional special space with a rectangular cross sectional shape in to the direction of the gas flow. A height of a cavity inside the sampling chamber may be typically 10-15 mm and a width of the cavity may be 5-15 mm. One or more optical windows 35, used for the gas concentration or component measurement based on for example an absorption of an infrared radiation by different gases, are conventionally located on one side or both sides of the airway adapter 3. This kind of construction provides a place for a sensor 40 to be located on a top of the sampling chamber 31 of airway adapter 3 according to this embodiment, which is then used for measuring the content of oxygen, or other gases as well, in the breathing gas mixture. Consequently the airway adapter 3 and said sensor 40 are integrated together to form an integral structure.

The sensor 40 may be a chemical cell, electro-chemical cell or similar that produces an electrical signal, voltage or current proportional to oxygen content of the measured gas mixture. The sensor 40 may be used to measure gases other than oxygen such as carbon dioxide or such as well. The size of the sensor 40 should be small, such that it can be firmly integrated or molded into the airway adapter 3 as also shown in the schematic side view in fig 2. The small size is achieved for example by decreasing a volume of the electrolyte inside the chemical -, or electro-chemical or similar sensor 40. A lifetime of the sensor 40 decreases proportional to the decreased volume of the electrolyte. On the contrary as the small sized sensor is integrated in to the airway adapter 3 it may be disposable rather than non-disposable, since airway adapters are conventionally hard to clean and they are replaced often with new ones. Typically one airway adapter is used with one subject only and during a longer period of a care it is changed typically every 1-3 days to prevent bacteria etc. that grows in airway adapters to harm the subject. Thus the typical lifetime of the sensor 40 according to this embodiment may be one week or less to achieve very small size. As the lifetime is decreased the sensor 40 also becomes more stable as different time drifts are decreased. The overall cost of the combined airway adapter 3 including the sensor 40 can be decreased by manufacturing disposable sensors in big quantities in a fully automated process.

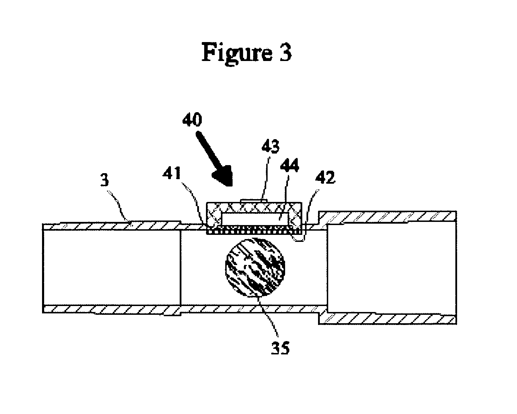

The connection between the sensor 40 and the airway adapter 3 is hermetic and gas tight to ensure there is no breathing gas leakages through the airway adapter. The hermetic connection is achieved by gluing, molding or similarly attaching the sensor 40 into the airway adapter 3. The sensor 40 comprising an electrolyte 44 as shown in Figure 3, which is reactive and sensitive to a measured gas or gases. The electrolyte 44 may be solid, gel or liquid comprising a separate film or coating or similar that forms a surface 41 that holds the electrolyte inside the sensor 40. The electrolyte 44 may also comprise electrical functions such as cathode and anode and/or chemical functions or chemicals. The surface 41 is preferably in straight contact with the breathing gas flowing inside the airway adapter 3 to ensure fast movement of gas molecules that pass the surface 41 into the sensor 40 thus ensuring a fast response time for the gas measurement. The surface 41 in contact with said respiratory gas is a basis for an analysis. When gas molecules of the respiratory gas flow are in contact with the surface 41 and through this surface with the electrolyte 44 an electrical signal is generated having a magnitude proportional to a quantity of particular gas molecules in the respiratory gas flow.

As shown in Figure 3 the sensor 40 may comprise an additional membrane 42, which is porous membrane close to or in straight contact with the surface 41 to prevent mucus etc. to clock the surface 41, which may prevent gas molecules to traverse through the membrane in to the surface 41 and into the sensor 40 sensitive to the measured gas or gases. The sensor 40 also comprises at least one electrical connection 43 for the voltage, current, resistance, capacitance etc. proportional to the measured gas content of the gas mixture flowing through the airway adapter 3. One or more electrical connections 43 are located on one or more sides of the sensor 40 that are outside airway adapter 3, but preferably they are on the top surface of the sensor 40 outside the airway adapter 3 as shown in fig. 3. The electrical connection 43 can also lie on a surface of the airway adapter 3.

When the gas analyzer 2 comprising an infrared emitter 11 for emitting an infrared radiation through the sampling chamber 31 and a detector 12 for receiving the infrared radiation and creating an infrared absorption signal is used for measuring the gas content or gas composition of the subject, the airway adapter 3 is placed into a coupling point 21 such as a cavity of the gas analyzer 2 so that breathing gases flowing through the endotracheal tube 4 and the branching unit 7 and through sampling chamber 31 in airway adapter 3 can be analyzed by the gas analyzer 2. As the airway adapter 3 is placed into the coupling point 21 of gas analyzer 2 preferably detachably one or more electrical connections 43 of the sensor 40 connect electrically to respective at least one electrical connection 22 of the coupling point 21 of the gas analyzer 2 shown in Figure 1. Through these electrical connections 22, 43 an electrical signal proportional to the measured gas content in the gas mixture of the breathing gas is transferred for further processing in to the processor 99 of the gas analyzer 2 which may comprise an amplifier (not shown in Figure 1), an analog to digital converter (not shown in Figure 1), a memory (not shown in Figure 1) etc. to get a calculated concentration of the measured gas or to get an identification of a gas component of the respiratory gas flow. These analyzing results including this calculated concentration information and/or the analyzed gas component information, if needed, further be received by a host device 50 such as a monitor e.g. for further processing or only for displaying the information. Alternatively the electrical signal proportional to the measured gas content in the gas mixture of the breathing gas may be transferred from the sensor 40 to the host device 50 for further processing to get a calculated concentration of the measured gas. Also this is same with the gas composition or identification signal.

Also processing of the signal as shown in Figure 2 may be made in the processor 99 of the airway adapter 3 comprising the amplifier 100, the analog to digital converter 101, the memory 102, the radio frequency transceiver 103 etc. and a small battery 104 that produces an electrical energy for the operation. The processor 99 with all these components may be integrated into the sensor 40, but around the airway adapter 3 as well (not shown in figures). As the airway adapter 3 and the sensor 40 integrated into one piece are preferably disposable rather than reusable and as the aim is to have very small sized combination of the sensor 40 and the airway adapter 3 as well as the low overall cost it may be more convenient to do further signal processing elsewhere. However low cost electronics including the processor 99 and the radio frequency transceiver 103 etc. as well as the small battery 104 may be produced with a very low cost applying new printed electronics techniques in which electrical components and functions are printed in to a plastic or paper type material with an electrically conductive polymer or polyaniline as printed ink designed specially for that purpose. A piece of flexible plastic made with printed electronics techniques may be wrapped around the airway adapter 3or as the adapter is preferably made of plastic or similar material the airway adapter 3 itself may even comprise the complete electronics and power source molded inside or around it. As shown in Figure 2 there is a wireless connection between the airway adapter 3 and the host device 50 for same reasons as discussed with Figure 1, but naturally the connection can be arranged through a wire, too.

The embodiments discussed hereinbefore solve many problems relating to conventional mainstream measurement of gases based on the chemical, electro-chemical or similar sensor. In the anesthesia and transportation the need for monitoring the same subject is normally hours. In intensive care the subject is usually monitored much longer time up to months, but during that period of time the breathing circuit or parts of it are often replaced several times, once every 1-3 days, as they get contaminated of mucus, bacteria, viruses etc. A shorter lifetime and a disposability of the gas sensor enables the miniaturization of conventional gas sensors to be merged into for example conventional airway adapters. This is possible for example by degreasing the amount of the electrolyte stored in to the sensor so that it corresponds to the shortened lifetime of the disposable sensor. The shorter usage time and a reasonable over dimensioning of reactive parts of the sensor make the sensor less sensitive to long-term signal drifts etc. during the shortened time period reducing or even eliminating the need for the calibration and zeroing.

According to the embodiments discussed hereinbefore the sensor 40 is sealed hermetically into the airway adapter 3, which eliminates the risk of the breathing gas leakage and the possibility of contaminating the non-disposable parts of the gas analyzer 2 thus preventing dangerous bacteria and viruses etc. to sift other subjects. The disposable measurement degreases a manual and time consuming cleaning work, but most of it improves the patient safety. Furthermore using packaging techniques and mass production to make the sensor cell small and inexpensive it reduces the cost of the whole measurement. Furthermore the surface 41 through which the gas molecules diffuse straight into the electrolyte 44 of the sensor 40 is placed directly into the breathing gas flowing inside the airway adapter 3, which degreases the response time of the gas measurement considerably. In the terms of rise time Xᵣ = Yᵣ + Zᵣ the rise time of the molecule diffusion Yᵣ is close to zero thus the rise time of the measurement Xᵣ is determined mainly by the rise time of the sensor Zᵣ, which is less than 300 ms for the new type of the sensor (40) described in this embodiment. Similarly in the terms of fall time X_{f} = Y_{f} + Z_{f} the fall time of the molecule diffusion Y_{f} is close to zero thus the fall time of the measurement is determined mainly by the fall time of the sensor Z_{f}, which is also less than 300 ms. In the terms of the respiration rate RR it is possible to measure RR more than 60 rpm (respiration per minute), depending mainly of the response time of the sensor, which is usually enough for the most of the mechanically ventilated subjects. One or more electrical connections 43 of the sensor 40 in airway adapter 3 connect to respective one or more electrical connections 22 of the gas analyzer 2 automatically as the airway adapter 3 is pressed in to the coupling point 21 of the gas analyzer 2 thus making the device easy to use also.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An airway adapter comprising:
a sampling chamber (31) for allowing a respiratory gas flow;
**characterized in that** a said airway adapter further comprising a sensor (40) for acquiring a signal needed for respiratory gas analysis,
wherein said sensor (40) is integrated into said airway adapter and which sensor comprises an electrolyte (44) being in contact with said respiratory gas flowing in said sampling chamber and which electrolyte contact with said respiratory gas is a basis for respiratory gas analysis.

2. The airway adapter according to claim 1, **characterized in that** said electrolyte of said sensor (40) has a surface (41) whereupon predetermined gas molecules in the respiratory gas flow pass said surface being in contact with said electrolyte and generate an electrical signal having a magnitude proportional to a quantity of particular gas molecules in the respiratory gas flow.

3. The airway adapter according to claim 1, **characterized in that** said sensor (40) further comprising an electrical connection (43) being connectable through this electrical connection to a gas analyzer (2) for making one of a qualitative or quantitative gas analysis of at least one respiratory gas like CO₂ or Oxygen.

4. The airway adapter according to claim 3, **characterized in that** said gas analyzer (2) comprises at least one electrical connection (22) and a coupling point (21) allowing said airway adapter (3) to be detachably placed into said coupling point (21), whereupon said electrical connection (22) of said gas analyzer (2) is in contact with said electrical connection (43) of said sensor (40).

5. The airway adapter according to claim 1, **characterized in that** said sensor (40) further comprising a membrane (42) between said electrolyte (44) and said sampling chamber (31) to keep said electrolyte functioning.

6. The airway adapter according to claim 1, **characterized in that** said airway adapter further comprising of an optical window (35) used while making an optical gas measurement of the respiratory gas flow.

7. The airway adapter according to claim 6, **characterized in that** said optical gas measurement is made by a gas analyzer (2) connectable to said airway adapter and that said gas analyzer (2) comprises an infrared emitter (11) and detector (12) for said optical gas measurement and that said gas analyzer (2) is connectable to a host device (50) for further exploiting said analysis made by said gas analyzer (2).

8. The airway sensor according to claim 7, **characterized in that** said gas analyzer (2) is making based on said optical gas measurement an analysis of at least one respiratory gas like CO₂ N₂O or anesthetic agent.

9. The airway adapter according to claim 1, further comprising a processor (99) for analyzing said signal received from said sensor (40) and that said processor (99) is configured to be in contact with a host device (50) receiving analyzing results.

10. A gas analysis unit for respiratory gas analysis comprising:
an airway adapter (3) having a sampling chamber (31) for allowing a respiratory gas flow;
a sensor (40) for acquiring a signal relative to the respiratory gas flowing through said sampling chamber (31) of said airway adapter (3); and
a processor (99) for analyzing said signal received from said sensor (40),
**characterized in that** said sensor (40) is integrated into said airway adapter (3) and which sensor comprises an electrolyte (44) being in contact with said respiratory gas in said sampling chamber (31) and which electrolyte contact with said respiratory gas is a basis for the respiratory gas analysis.

11. The gas analysis unit according to claim 10, **characterized in that** said processor (99) is integrated with one of said airway adapter (3) and said sensor (40).

12. The gas analysis unit according to claim 10, **characterized in that** said processor (99) may comprise an amplifier (100), a digital converter (101), a memory (102), a radio frequency transceiver (103) and a battery (104).

13. The gas analysis unit according to claim 10, **characterized in that** said signal relative to the respiratory gas is indicative of a concentration of a gas component and/or is indicative of a gas component included in the respiratory gas.

14. A gas analysis unit for respiratory gas analysis comprising:
an airway adapter (3) having a sampling chamber (31) for allowing a respiratory gas flow and at least one optical window (35) for an optical measurement of the respiratory gas;
a sensor (40) for acquiring a signal indicative of one of a concentration of a component and an identification of a component of the respiratory gas flowing through said sampling chamber (31) of said airway adapter (3); and
a gas analyzer (2) for receiving said signal from said sensor (40), said gas analyzer (2) comprising a coupling point (21) for an airway adapter (3), an infrared emitter (11) for emitting an infrared radiation through said sampling chamber and a detector (12) for receiving the infrared radiation and creating an infrared absorption signal and a processor (99) for analyzing said signal from said sensor (40) and said infrared absorption signal from said detector (12),
**characterized in that** said sensor (40) is integrated into said airway adapter (3) and which sensor comprises an electrolyte (44) being in contact with said respiratory gas in said sampling chamber (31) and which electrolyte contact with said respiratory gas is a basis for respiratory gas analysis.

15. The gas analysis unit according to claim 14, **characterized in that** said gas analyzer (2) is detachable and that said processor (99) is configured to be in contact with a host device (50) receiving analyzing results.
